# EUROPÄISCHE PATENTSCHRIFT

(11) **EP 2 731 706 B1**
(45) Veröffentlichungstag und Bekanntmachung des Hinweises auf die Patenterteilung: **09.06.2021**
(21) Anmeldenummer: 13713880.6
(22) Anmeldetag: 02.04.2013
(51) Int. Cl.: B01J 20/18, G21F 9/02, B01D 53/82, B01D 53/70, B01D 53/02, B01D 53/04, B01J 20/02, B01J 20/28

(54) **METHYLIODIDADSORBER, VERWENDUNG DESSELBEN UND VERFAHREN ZUR ADSORPTION VON METHYLIODID**
ADSORBER FOR METHYLIODIDE, USE OF IT AND METHOD FOR ADSORPTION OF METHYLIODIDE
ADSORBEUR DE IODURE DE MÉTHYLE, SON UTILISATION ET PROCÉDÉ POUR L'ADSORPTION DE IODURE DE MÉTHYLE

(30) Priorität: 02.04.2012 DE 102012006542
(43) Veröffentlichungstag der Anmeldung: 21.05.2014
(73) Patentinhaber: Clariant International Ltd, 4132 Muttenz (CH); Framatome GmbH, 91052 Erlangen (DE)
(72) Erfinder: DONABAUER, Katharina, 83052 Bruckmühl (DE); TISSLER, Arno, 93105 Tegernheim (DE); ENDLER, Mika, 83024 Rosenheim (DE); BÜTTNER, Olaf, 83059 Kolbermoor (DE)
(74) Vertreter: Kuba, Stefan
(86) Internationale Anmeldenummer: PCT/EP2013/056941
(87) Internationale Veröffentlichungsnummer: WO 2013/150029

(56) Entgegenhaltungen:
- EP-A1- 0 175 435
- EP-A1- 0 379 895
- EP-A2- 2 423 923
- CN-A- 101 811 023
- CN-A- 101 829 542
- US-A- 3 838 554
- US-A- 4 382 879

## Beschreibung

Die vorliegende Erfindung betrifft einen Methyliodidadsorber, eine Verwendung desselben und ein Verfahren zur Adsorption von Methyliodid.

In Kernkraftwerken werden alle Bauteile, die mit radioaktiven Stoffen in Berührung kommen, in der sogenannten "Nuklearen Insel" (Nuclear Island) zusammengefasst. Diese umfasst den Sicherheitsbehälter (Inneres Containment) mit Primärkreislauf, dem Flutbecken und dem Kernfänger. Im oberen Teil des Sicherheitsbehälters können katalytische Rekombinatoren bzw. Rekombinatorsysteme, beispielsweise mit Pd auf Al₂O₃, installiert sein, die den Wasserstoffanteil in der Atmosphäre begrenzen sollen, um Wasserstoffexplosionen zu verhindern.

In Kernspaltungsreaktoren treten im Normalbetrieb, aber insbesondere im Störfall neben den festen Zerfallsisotopen des Urans bzw. aus der Spaltung des Urans hervorgegangenen Tochterisotopen und durch Neutroneneinfang entstandenen höheren Isotopen, auch gasförmige radioaktive Verbindungen auf, deren Freisetzung in die Umwelt unbedingt vermieden werden muss. Die wichtigsten hierbei sind neben Wasserstoff radioaktives Iod und Methyliodid.

Wasserstoff entsteht in geringen Mengen im Normalbetrieb und in großen Mengen bei Störfällen, die mit einer starken Temperaturerhöhung verbunden sind, durch die Reaktion des Wassers mit der Metallhülle der Kernbrennstäbe. Dieser Wasserstoff kann dann in einer Knallgasreaktion zur Zerstörung des Sicherheitsbehälters und zur Freisetzung von großen Mengen an radioaktivem Material führen (z.B. die Unfälle von Tschernobyl und Fukushima). Um derartige Katastrophen zu vermeiden, werden in letzter Zeit Kernkraftwerke mit Rekombinator-Systemen ausgestattet bzw. nachgerüstet. Dabei handelt es sich um passive Systeme, deren Aufgabe es ist, bei Raumtemperatur unter Atmosphärenbedingungen gebildeten Wasserstoff katalytisch wieder zu Wasserdampf zu oxidieren und so die Entstehung explosiver Atmosphären zu vermeiden. Auch für Abklingbecken und Brennelementbehälter können Rekombinatoren verwendet werden, die dafür sorgen, dass der freigesetzte Wasserstoff zu Wasser abreagieren kann, bevor es zu einer explosiven Konzentration kommt.

Aus den Spaltprodukten Cäsium und Iod entsteht in den Kernbrennstäben auch Cäsiumiodid, das sich, soweit nicht in den Brennstäben zurückgehalten, im Reaktorsumpf sammelt. Aus Cäsiumiodid bildet sich im Reaktorsumpf durch Radiolyse oder an heißen Stellen (z.B. an Hotspots in Wasserstoffrekombinatoren) elementares Iod, das durch seine Flüchtigkeit auch im Normalbetrieb aus dem Reaktorsumpf austreten kann. Freies Iod kann dann durch seine Reaktivität mit organischen Substanzen aus der Reaktorumgebung (z.B. Farbe) zu Methyliodid reagieren. Diese flüchtigen radioaktiven Substanzen reichern sich im Gasinventar innerhalb der Reaktorhülle an und müssen von dort adsorbiert werden. Viele der gebildeten Verbindungen liegen auch Aerosol-gebunden vor und werden bei einem Störfall freigesetzt.

Ein Großteil der gebildeten radioaktiven Iod-Isotope weist eine kurze Halbwertszeit auf und trägt damit durch die hohe radiologische Aktivität im Falle eines Störfalls sehr stark zur Gefährdung von Lebewesen bei. Iod lagert sich in der Schilddrüse an und verursacht bei hohen Konzentrationen Schilddrüsenkrebs. Insbesondere Iod 131 mit einer Halbwertszeit von 8 Tagen ist hier zu nennen.

Es besteht daher ein Bedarf an auch unter hoher Feuchtigkeit stabilen Adsorbern für radioaktives Methyliodid, zur Ausrüstung von Kernreaktoren bzw. zur Umrüstung in den sicheren Betrieb oder zur Sicherung des Reaktors im Stillstand oder bei bzw. nach der Stilllegung.

Auf Grund von neuen Erkenntnissen und Sicherheitsbestimmungen ist die Zurückhaltung von organischem Methyliodid zur aktuellen Aufgabe geworden, was auch eine Lehre aus dem Vorfall in Fukushima im Jahre 2011 ist. Eine technische Lösung gab es bisher noch nicht. Es wurden aber verschiedene Ansätze verfolgt. Adsorbentien zur Abscheidung von elementarem Iod sind bekannt, wie z.B. mit Silber beladene Aluminiumoxide. Diese sind unter Wasserdampf durch Inhibierung jedoch wenig nutzbar.

Für die Abscheidung von Methyliodid gab es bisher noch keine funktionierende technisch ausgereifte Maßnahme. Bekannt ist, dass Silber-haltige Adsorbentien für die Adsorption von Methyliodid geeignet sind. Das gebildete Silberiodid besitzt einen Schmelzpunkt von ca. 600°C und einen Siedepunkt von ca. 1500°C, ist also unter Normalbedingungen weitgehend stabil. Bei bekannten Adsorbentien kann sich jedoch unter hohen Wasserdampfkonzentrationen Wasser in den porösen Strukturen des Adsorbers einlagern und damit die Adsorption von Methyliodid inhibieren. Eine Möglichkeit, diese Inhibierung zu vermeiden, besteht in einer Silanisierung der äußeren Adsorberoberfläche durch organische Silanverbindungen. Diese Methode ist teuer und weist schwerwiegende technische Probleme auf. Die Silan-Schicht zersetzt sich ab ca. 180°C unter starker Hitzeentwicklung. Dies bedeutet, dass ab dieser Temperatur keine Adsorption von Methyliodid mehr stattfinden kann und weiter, dass durch die starke Exothermie möglicherweise vorhandener Wasserstoff gezündet wird, was zu den ungewünschten Begleiterscheinungen einer Explosion führen kann.

Aufgabe war es daher, ein Adsorbens zur Verfügung zu stellen, mit dem die vorgenannten Nachteile vermieden werden und das sich zudem durch eine hohe Stabilität und eine hohe Iodid-Adsorptionskapazität auszeichnet.

Diese Aufgabe wird gelöst durch einen Methyliodidadsorber nach Anspruch 1, eine Verwendung desselben nach Anspruch 8 und ein Verfahren zur Adsorption von Methyliodid nach Anspruch 10 (Ausführungsformen).

Überraschenderweise ist der Adsorber nach den Ausführungsformen stark hydrophob, zeigt eine hohe Iodid-Adsorptionskapazität und ist über einen weiten Bereich der Wasserdampfkonzentration und der Temperatur in diesen Eigenschaften stabil. Weiterhin ist die Gefahr einer exothermen Zündung von eventuell vorhandenem Wasserstoff durch die hydrophoben Eigenschaften und die Abwesenheit einer möglicherweise unter Exothermie desorbierenden Silan-Schicht deutlich verringert.

Weitere Merkmale und Zweckmäßigkeiten ergeben sich aus der folgenden Beschreibung von Ausführungsformen, den Figuren und den Unteransprüchen.

Alle hier beschriebenen und sich nicht gegenseitig ausschließenden Merkmale von Ausführungsformen können miteinander kombiniert werden. Elemente einer Ausführungsform können in den anderen Ausführungsformen genutzt werden ohne weitere Erwähnung. Ausführungsformen der Erfindung werden nun durch die nachfolgenden Beispiele genauer beschrieben, ohne sie dadurch einschränken zu wollen.

In der nachfolgenden Beschreibung von Ausführungsformen wird der Methyliodidadsorber auch einfach Adsorber genannt. Zudem werden in der folgenden Beschreibung die Begriffe Zeolith und Zeolithmaterial synonym verwendet.
Silber, das in den Ausführungsformen als das Iodid adsorbierende Metall eingesetzt wird, reagiert mit Methyliodid unter Ausbildung von Silberiodid und bindet somit das Methyliodid im Adsorber durch Chemisorption.

Der Begriff "umfassend" beinhaltet in Ausführungsformen "im Wesentlichen bestehend aus" oder "bestehend aus" und kann durch diese ersetzt werden. Für grammatikalische Abwandlungen des Wortes "umfassend" gilt dies entsprechend. Ferner gilt hier für die Beschreibung von Wertebereichen, dass die Angabe eines breiten Bereichs mit engeren alternativen oder bevorzugten Bereichen auch Bereiche offenbart, die durch eine beliebige Kombination angegebener unterer Bereichsgrenzen mit angegebenen oberen Bereichsgrenzen gebildet werden können.

In den Ausführungsformen enthält der Methyliodidadsorber Silber als Iodid adsorbierendes Metall oder eine Verbindung davon., der Adsorber kann dabei auch elementares Jod, z.B. mit Adsorptionsraten größer 99%, adsorbieren.

Das Iodid adsorbierende Metall ist Silber, der Methyliodidadsorber kann aber beispielsweisedarüber hinaus Yttrium, Cer, Magnesium, Natrium, Zinn und Blei enthalten. In Ausführungsformen können auch mehrere dieser Iodid adsorbierenden Metalle neben Silber eingesetzt werden. Ferner kann neben Silber eines oder mehrere der Iodid adsorbierenden Metalle in seiner/ihrer kationischen Form enthalten sein. Durch die Verwendung von Silber als das Iodid adsorbierende Metall ist der Dampfdruck des entstandenen Silberiodids mit einem Siedepunkt von 1500°C sehr niedrig und das radioaktive Iodid somit sicher im Adsorber gebunden.

Wird bei einer Kernschmelze eine Temperatur von höher als ca. 1200°C erreicht, wird aus dem Adsorber von Ausführungsformen das radioaktive Iodid nicht freigesetzt, sondern durch die Umwandlung des Zeolithen in eine thermodynamisch stabilere Struktur (Cristobalit bzw. Quarz) im eigentlichen Sinn des Wortes verglast und ist damit bis zu einem Aufschmelzen im Quarz bzw. im Cristobalit bis zu Temperaturen von ca. 1700°C sicher verwahrt.

Wie oben erläutert, ist der erfindungsgemäße Methyliodidadsorber hydrophob und zeigt zudem überraschenderweise eine ausgeprägte Adsorptionsfähigkeit für Iodid. Letztere kann in Ausführungsformen zu einer Iodid-Abscheidung von über 99% führen. Ferner ist der erfindungsgemäße Methyliodidadsorber über einen weiten Bereich der Wasserdampfkonzentration und der Temperatur in diesen Eigenschaften stabil. Insbesondere ist die Hydrophobizität, d.h. der Ausprägungsgrad der Hydrophobie, selbst bei hohen Temperaturen stabil. Weiterhin ist die Gefahr einer exothermen Zündung von eventuell vorhandenem Wasserstoff durch die hydrophoben Eigenschaften deutlich verringert. Diese Effekte sind zum Beispiel dann zu beobachten, wenn bereits der bei der Herstellung eingesetzte, nicht mit Silber beladene bzw. undotierte Zeolith an sich hydrophob ist, d.h. wenn er eine hohe "intrinsische" Hydrophobie besitzt. In einigen Beispielen kann die Hydrophobie des Zeolithen und/oder des Adsorbers darüber hinaus auch bei hohen Temperaturen bis zur Zerstörung der Zeolithstruktur oberhalb von 1000°C erhalten bleiben.

Ferner sind aufgrund der hydrophoben Eigenschaften des in Ausführungsformen eingesetzten Zeolithen keine Beschichtung, z.B. mit siliziumorganischen Verbindungen, oder andere Maßnahmen erforderlich, um den mit Silber beladenen Zeolithen bzw. den Adsorber hydrophob zu machen. Dies hat zur Folge, dass eine Regeneration des Adsorbers durch Erhitzen ermöglicht wird. Die hydrophoben Eigenschaften des Zeolithen werden in Ausführungsformen der Erfindung nämlich nicht durch hohe Temperaturen beeinträchtig. Außerdem wird die Menge der dabei erzeugten Zersetzungsprodukte im Vergleich zu Adsorbern, die hydrophobe Beschichtungen aufweisen, deutlich reduziert.

Unter einem Zeolithen bzw. einem Zeolithmaterial wird im Rahmen der vorliegenden Erfindung gemäß einer Definition der International Mineralogical Association (D.S. Coombs et al., Can. Mineralogist, 35, 1997, 1571) eine kristalline Substanz mit einer durch ein Gerüst aus miteinander verbundenen Tetraedern charakterisierten Struktur verstanden. Dabei besteht jedes Tetraeder aus vier Sauerstoffatomen, die ein Zentralatom umgeben, wobei das Gerüst offene Hohlräume in Form von Kanälen und Käfigen enthält, die normalerweise von Wassermolekülen und Extragerüstkationen, welche ausgetauscht werden können, besetzt sind. Die Kanäle des Materials sind dabei groß genug, um Gastverbindungen den Zugang zu erlauben. Bei den hydratisierten Materialien erfolgt die Dehydratisierung meistens bei Temperaturen unterhalb von etwa 400 °C und ist zum größten Teil reversibel.

Der Zeolith, der in den Methyliodidadsorbern nach den Ausführungsformen enthalten ist, ist ein hydrophober Zeolith vom Strukturtyp BEA und weist ein SiO₂/Al₂O₃-Verhältnis > 100 auf.

Es hat sich überraschend herausgestellt, dass erst ein hohes SiO₂/Al₂O₃-Verhältnis größer 100 ausreichend hydrophobe Eigenschaften des mit Silber beladenen Zeolithen, wie auch des gesamten Adsorbers bewirkt. Gemäß einigen Ausführungsformen liegt das SiO₂/Al₂O₃-Verhältnis des verwendeten Zeolithen im Bereich > 140, z.B. zwischen 100 und 250 oder zwischen 130 und 170.

Die vorstehende Dreibuchstabencode-Nomenklatur BEA entspricht dabei der "IUPAC Commission of Zeolite Nomenclature".

Ferner kann der Adsorber, der den Zeolithen enthält als Formkörper ausbildet sein, oder als Washcoat, der den Zeolithen enthält und der auf einem Träger angeordnet ist. Der Anteil an Mikroporen ist hierbei mehr als 70 %, bevorzugt mehr als 80 %, bezogen auf das Gesamtporenvolumen des Zeolithen oder Adsorbers. Dabei sollen im Rahmen der vorliegenden Erfindung unter dem Begriffen Mikroporen, Mesoporen und Makroporen Poren verstanden werden, die einen Durchmesser von < 1 Nanometer (Mikroporen), einen Durchmesser von 1 bis 50 Nanometer (Mesoporen), beziehungsweise einen Durchmesser von > 50 Nanometer (Makroporen) aufweisen.

Die Mikroporosität des Zeolithen gemäß Ausführungsformen bewirkt eine starke und stabile Dispersität des Silbers im Zeolithen, denn durch die mikroporöse und uniforme Porenstruktur des Zeolithen wird das Silber stark in die Dispersion gezwungen und ist somit gleichmäßig im Zeolithen verteilt. Dies fördert zudem die Aufnahme von Iodid in den Zeolithen, denn im Vergleich zu ähnlich beladenen anderen Strukturen, wie z.B. Aluminiumoxid, zeigt der Zeolith von Ausführungsformen eine erhöhte Iodid-Adsorptionskapazität. Zusätzlich ist in Ausführungsformen der Dampfdruck des Silbers, das Iodid adsorbiert hat, durch die mikroporöse Struktur erniedrigt. Durch die Verwendung von Silber als das Iodid adsorbierende Metall ist der Dampfdruck des entstandenen Silberiodids mit einem Siedepunkt von 1500°C sehr niedrig. Wird bei einer Kernschmelze eine Temperatur von höher als ca. 1200°C erreicht, wird zudem das radioaktive Iodid nicht freigesetzt, sondern durch die Umwandlung des Zeolithen in eine thermodynamisch stabilere Struktur (Cristobalit bzw. Quarz) im eigentlichen Sinn des Wortes verglast und ist damit bis zu einem Aufschmelzen im Quarz bzw. im Cristobalit bis zu Temperaturen von ca. 1700°C sicher verwahrt.

Die Anwesenheit des Silbers im Wesentlichen in den Poren des Zeolithen, führt ebenfalls zu einer starken und stabilen Dispersität des Silbers im Zeolithen, denn so wird das Silber zusätzlich stark in die Dispersion gezwungen und ist somit gleichmäßig im Zeolithen verteilt. Dies fördert zudem die Aufnahme von Iodid in den Zeolithen. Ferner wird dadurch eine Agglomeration des Silbers zu größeren Partikeln bei hohen Temperaturen deutlich verlangsamt oder verhindert, die zu einem Verlust an wirksamer Oberfläche und damit an Performance führen würde.

Das Silber, muss erfindungsgemäß durch Incipient Wetness-Imprägnation in den Zeolithen eingebracht sein. Das Silber kann in Form von Partikeln im Zeolithen vorliegen. Desweiteren können die Partikel einen mittleren Durchmesser von 0,5 bis 5 nm, vorzugsweise 0,5 - 1,5 nm aufweisen und sind damit vorzugsweise XRD-amorph.

Der Adsorber von Ausführungsformen kann mindestens einen Promotor zur Verringerung der Wasserzersetzung durch Silber enthalten. Da das Iodid adsorbierende Metall Silber ist, kann unter erhöhten Temperaturen und Wasserdampfkonzentrationen nämlich eine katalytische Zersetzung des Wassers zu Wasserstoff und Sauerstoff auftreten. Dieses Problem wird gemäß Ausführungsformen durch die Dotierung des Methyliodidadsorbers mit dem Promotor gelöst.

Der Promotor kann beispielsweise Blei (Pb) umfassen. Durch den Promotor wird die Iodid-Adsorptionskapazität nicht erniedrigt, vielmehr wird die Neigung des Silbers zur Wasserzersetzung reduziert und damit die Bildung des Wasserstoffgases vermieden.

Der Gehalt an Silber im Zeolithen beträgt 5 bis 30 Gew.-% bevorzugt 10 bis 20 Gew.-%. Ferner kann der Gehalt an Promotor oder Blei im Zeolithen oder im Adsorber 1 bis 30 Gew.-%, mehr bevorzugt 5 bis 30 Gew.-%, weiter bevorzugt 10 bis 20 Gew.-%, und am meisten bevorzugt 3 bis 6 Gew.-% betragen.

Die BET-Oberfläche des Zeolithen oder des Adsorbers von Ausführungsbeispielen kann 10 bis 1000 m²/g, bevorzugt 300 bis 900 m²/g, besonders bevorzugt 500 bis 700 m²/g betragen und/oder das integrale Porenvolumen des Zeolithen oder des Adsorbers von Ausführungsbeispielen kann größer als 100 mm³/g, bevorzugt größer als 200 mm³/g sein. Durch diese Eigenschaften einzeln oder in Kombination kann die Adsorptionskapazität des Adsorbers günstig beeinflusst werden.

Der Adsorber von Ausführungsbeispielen kann als Schüttgut ausgebildet sein. Der Adsorber kann ferner beispielsweise als Extrudat, als Formkörper oder als mit dem Zeolithen beschichteter Partikel ausgebildet sein. Zum Beispiel kann das Schüttgut aus Formkörpern oder Pellets bestehen, die durch Pressen oder Extrusion einer keramischen Masse des mit Silber beladenen Zeolithen erzeugt worden sind.

Als beispielhafte geometrische Form des Adsorbers bzw. der Formkörper sind Kugeln, Ringe, Zylinder, Lochzylinder, Triloben oder Kegel, wobei besonders bevorzugt ein Monolith ist, beispielsweise ein monolithischer Wabenkörper, zu erwähnen.

Ferner kann der Adsorber einen Träger umfassen, auf dem der Zeolith oder ein den Zeolithen enthaltender Washcoat aufgebracht ist. Als Washcoat dient z.B. eine Suspension oder eine Aufschlämmung des Zeolithen in einem Suspensionsmittel, z.B. in Wasser, ggf. unter Zusatz eines bevorzugt silikatischen Binders. Der Zeolith kann beispielsweise durch Beschichten mit einer Suspension bzw. mit dem Washcoat oder durch Aufwachsen aus einer Lösung auf den Träger aufgebracht werden.

Der Adsorber und/oder der Träger kann wabenförmig oder plattenförmig, z.B. als Bleche, ausgebildet sein. Die plattenförmige Variante ermöglicht eine parallele Installation mehrerer Adsorber im oberen Bereich der Sicherheitsbehälter von Kernkraftwerken, wodurch eine gute Durchströmung der Adsorber mit dem Methyliodid enthaltenden Gas erzielt werden kann.

In Verbindung mit einem Washcoat ist es darüber hinaus bevorzugt, wenn der Adsorber, sofern er wabenförmig ausgeprägt ist, eine Beladung mit dem Silber von 0,1 bis 4,0 g/l, mehr bevorzugt 0,4 bis 1,5 g/l, und am meisten bevorzugt 0,4 bis 1,0 g/l, bezogen auf das Volumen des Wabenkörpers enthält.

In Ausführungsformen kann der Träger als Trägermaterial ein Metalloxid umfassen, vorzugsweise ein Titanoxid, ein Ceroxid, ein Aluminiumoxid, ein Zinnoxid, ein Zirkoniumoxid, ein Siliziumoxid, ein Zinkoxid, ein Aluminiumoxid-Siliziumoxid oder ein Magnesiumsilikat oder eine Mischung von zwei oder mehr der vorgenannten Oxide. Es können Träger oder Tragkörper aus keramischem Material Verwendung finden. Häufig handelt es sich bei dem keramischen Material um ein intertes Niedrigoberflächenmaterial wie Cordierit, Mullit, alpha-Aluminiumoxid, Siliciumcarbid oder Aluminiumtitanat. Jedoch kann der eingesetzte Tragkörper auch aus hochoberflächigem Material wie gamma-Aluminiumoxid oder TiO₂ bestehen. Auch Metalle sind als Trägermaterial einsetzbar. Daher sind ebenfalls bevorzugte Träger bzw. Tragkörper beispielsweise aus einem Blech, aus einem beliebigen Metall oder einer Metalllegierung gebildet, die eine Metallfolie oder Sintermetallfolie oder ein Metallgewebe aufweisen und zum Beispiel durch Extrusion, Aufwickeln oder Stapeln hergestellt werden.

Desweiteren kann der Adsorber von Ausführungsformen zur Adsorption von Methyliodid, radioaktivem Methyliodid, Iod und/oder radioaktivem Iod verwendet werden. Dabei kann der Adsorber insbesondere in oder in der Nähe von Kernkraftwerken, Wiederaufbereitungsanlagen oder Brennelementlagern eingesetzt werden, z.B. in Sicherheitsbehältern oder Abklingbecken von Kernkraftwerken oder in Behältern für bestrahlte oder abgebrannte Brennelemente.

Außerdem kann der Adsorber zusammen mit einem Wasserstoffrekombinationskatalysator, auch Rekombinator genannt, eingesetzt werden. Dieser kann anstelle oder zusätzlich zu einem im Adsorber enthaltenen Promotor eingesetzt werden. Auf diese Weise kann unter erhöhten Temperaturen und/oder unter erhöhten Wasserdampfkonzentrationen eine durch das Silber katalysierte Zersetzung des Wassers zu Wasserstoff und Sauerstoff vermieden bzw. verhindert werden.

Eine weitere Ausführungsform betrifft ein Verfahren zur Adsorption von Methyliodid, bei dem Methyliodid mit einem Adsorber gemäß den vorgenannten Ausführungsformen in Kontakt gebracht wird. Zusätzlich zum Methyliodid kann auch Iod mit dem Adsorber gemäß den vorgenannten Ausführungsformen in Kontakt gebracht werden und von diesem adsorbiert werden. Das Methyliodid und/oder das Iod kann radioaktiv sein, insbesondere wenn das Verfahren in Kernkraftwerken, Wiederaufbereitungsanlagen oder Brennelementlagern eingesetzt wird.

Ein anderes Beispiel eines Verfahrens zur Herstellung des Adsorbers umfasst: a) Einbringen von Silber durch Incipient Wetness-Imprägnation in das Zeolithmaterial; b) Nassvermahlen des mit Silberverbindung beladenen Zeolithmaterials mit einem Trägermaterial, das porös sein kann; c) Trocknen des Gemisches umfassend das beladene Zeolithmaterial und das Trägermaterial. Dabei kann nach dem Schritt a) und vor dem Schritt b) ein Fixierungsschritt erfolgen, in welchem die Silberverbindung an dem Zeolithmaterial fixiert wird. Ferner kann ein Stabilisierungsschritt zur Stabilisierung des Adsorbers durchgeführt werden. Häufig wird als Trägermaterial ein Silica-Sol wie Bindzil verwendet, das flüssig ist und nach dem Trocknen porös ist. Auf diese Weise ist ein Adsorber erhältlich, umfassend ein poröses Trägermaterial sowie ein Zeolithmaterial, dessen innere Oberfläche mit dem Silber beladen ist.

Zum Beispiel kann das im Schritt b) erhaltene nasse Gemisch oder das im Schritt c) erhaltene getrocknete Gemisch in einer Aufschlämmung oder in einem Washcoat auf einen Träger aufgebracht werden. Unter Adsorbern können somit im Rahmen dieser Erfindung auch Adsorber verstanden werden, die durch Beschichten eines Trägerkörpers mit einer typischerweise porösen Schicht enthaltend Silber hergestellt werden.

In dem Verfahren zur Herstellung des erfindungsgemäßen Methyliodidadsorbers erfolgt das Einbringen des Silbers durch Imprägnieren des Zeolithmaterials mit einer Lösung der Metallverbindung mittels der Pore-filling-Methode (Incipient Wettness-Imprägnation). Dabei wird das Zeolithmaterial mit einer Menge an Lösung in Kontakt gebracht, deren Volumen dem Porenvolumen des eingesetzten Zeolithmaterials entspricht.

Als Verbindungen des Silbers oder des Promotors können im Herstellungsverfahren des Adsorbers die entsprechenden Nitrate, Acetate, Oxalate, Tartrate, Formeate, Amine, Sulfite, Carbonate, Halogenide oder Hydroxide eingesetzt werden.

Bei der Herstellung des Adsorbers gemäß Ausführungsformen muss das Zeolithmaterial ein mikroporöses Zeolithmaterial vom Strukturtyp BEA sein.

Bei dem Adsorber von Ausführungsformen kann es sich außerdem um einen Adsorber mit polymodaler Porenverteilung handeln, das heißt, er enthält sowohl Mikroporen, Mesoporen als auch Makroporen. Dabei sollen im Rahmen der vorliegenden Erfindung unter dem Begriffen Mikroporen, Mesoporen und Makroporen Poren verstanden werden, die einen Durchmesser von < 1 Nanometer (Mikroporen), einen Durchmesser von 1 bis 50 Nanometer (Mesoporen), beziehungsweise einen Durchmesser von > 50 Nanometer (Makroporen) aufweisen. Die Bestimmung des Mikro- und Meso-/Makroporenanteils wird mittels der sogenannten T-Plot Methode gemäß ASTM D-4365-85 durchgeführt.

Der Adsorber gemäß Ausführungsformen kann beispielsweise einen porösen SiO₂-haltigen Binder umfassen, wobei der Adsorber einen Anteil an Mikroporen, z.B. mit einem Durchmesser < 1 nm, von mehr als 70 %, bezogen auf das Gesamtporenvolumen des Adsorbers, aufweisen kann. Ferner kann das Zeolithmaterial einen Aluminiumanteil von weniger als 2 Mol-% besitzen. Das Gewichtsverhältnis Zeolithmaterial/Binder (bezogen auf die jeweiligen Trockenmassen) kann 99:1 bis 1:99 betragen. Als SiO₂-haltigen Binder kann ein reiner SiO₂-Binder, der wenig Meso-und Makroporen aufweist, eingesetzt werden, z.B. Bindzil 2034 DI-Suspension (Eka-Chemicals AB, Bohus/Schweden). Es wurde nämlich gefunden, dass Adsorber, die ein mikroporöses Zeolithmaterial, das ein Iodid adsorbierendes Metall enthält, und einen reinen SiO₂-Binder, der wenig Meso- und Makroporen aufweist, umfassen, eine deutlich höhere Adsorptionsfähigkeit für Iodid aufweisen.

Ein derartiger Adsorber von Ausführungsformen ist herstellbar durch a) Einbringen von Silber durch Incipient Wettness-Imprägnation in das mikroporöses Zeolithmaterial; b) Vermischen des so entstandenen, mit einer Silberverbindung beladenen Zeolithmaterials mit einem porösen SiO₂-haltigen Binder und einem Lösungsmittel; und c) Trocknen des Gemisches umfassend das mit der Silberverbindung beladene Zeolithmaterial und den Binder. Dabei kann die in Schritt b) erhaltene Mischung auf einen Träger, auch Tragkörper oder Trägerkörper genannt, aufgebracht werden.

In einem weiteren Verfahren zur Herstellung des Adsorbers von Ausführungsformen wird ein Adsorber erzeugt, der neben dem Silber einen metallhaltigen Promotor enthält, hier auch bimetallischer Adsorber genannt. Dieses Beispiel wird anhand der Herstellung eines Ag- und Pb-haltigen Adsorbers beschrieben, der erhalten werden kann durch: Imprägnieren eines Trägermaterials aus dem hydrophoben Zeolithen mit schwefelfreien Ag- und Pb-Verbindungen durch Incipient Wettness-Imprägnation, und Trocknen des imprägnierten zeolithischen Trägermaterials an Luft. Dabei können als Ag- und Pb-Verbindungen z.B. Lösungen der Nitrate verwendet werden. Das Trocknen des imprägnierten zeolithischen Trägermaterials kann bevorzugt unterhalb des Zersetzungspunktes der Ag- und Pb- Verbindungen durchgeführt werden. In diesem Verfahren können desweiteren die folgenden Schritte enthalten sein: Herstellen eines Washcoats aus dem imprägnierten zeolithischen Trägermaterial, Beschichten eines Trägerkörpers mit dem Washcoat, und Trocknen des beschichteten Trägerkörpers an Luft.

Auf diese Weise ist ein Adsorber gemäß Ausführungsformen herstellbar, wobei der Adsorber eine bimetallische Zusammensetzung enthaltend Ag und Pb auf einem zeolithischen Trägermaterial enthält. Die bimetallische Zusammensetzung kann dabei eine BET-Oberfläche von mehr als 400 m²/g aufweisen. Insbesondere kann die bimetallische Zusammensetzung oder die Washcoat-Beschichtung ein Ag/Pb-Gewichtsverhältnis von 6:1 bis 1:1 besitzen. Ag und Pb befindet sich im Adsorber dieses Beispiels im Wesentlichen in den Poren des zeolithischen Trägermaterials und liegt in Aggregaten von < 5 nm vor.

### Messmethoden

### Elementanalytik mit ICP:

Die ICP-AES (Inductively coupled plasma atomic emission spectroscopy) zur Ermittlung der Elementzusammensetzung bzw. des SiO₂/Al₂O₃-Verhältnisses wurde mit dem Gerät ICP Spectro Modula/Arcos durchgeführt. Als Chemikalien wurden dabei eingesetzt: Schwefelsäure 98% p.A., Flusssäure 37% p.A, Salzsäure 37% p.A. Die Probe war fein gemahlen.

Für Si und Al wurden 100 mg Probe in einen 100 ml Plastikbecher eingewogen und mit 1 ml Schwefelsäure und mit 4 ml Flusssäure versetzt. Im Wasserbad wurde 5 Min. lang bei 85°C aufgeschlossen bis eine klare Lösung entstand. Nun wurde temperiert, aufgefüllt und geschüttelt. Alle Elemente wurden am ICP gemessen, ebenso entsprechende Standards. Si wurde mit folgenden Einstellungen gemessen: Wellenlänge: 288, 158 nm. Al wurde gemessen mit folgenden Einstellungen: Wellenlänge: 396, 152 nm.

Für Ag und/oder Pb wurde soviel Probe eingewogen, dass sich ungefähr 3 mg Ag bzw. Pb darin befanden. Anschließend wurden jeweils 6 ml Flusssäure und Salzsäure zugegeben. Nun wurde unter Rühren 30 Min. lang auf 180°C erhitzt, um eine klare Lösung zu erzeugen. Nun wurde temperiert, aufgefüllt und geschüttelt. Alle Elemente wurden am ICP gemessen, ebenso entsprechende Standards. Ag wurde mit folgenden Einstellungen gemessen: Wellenlänge: 214, 423 nm. Für Pb beträgt die Wellenlänge: 168 nm.

Alle Standards waren angepasst mit HF und HCl bzw. H₂SO₄. Die Auswertung folgte folgender Berechnung: w(E* in Prozent) = ß(E*-Messwert in mg/l) x V(Messkolben in 1) x 100 / m(Einwaage in mg) (E*= jeweiliges Element).

### BET-Oberfläche:

Die Bestimmung erfolgt nach der BET-Methode gemäss DIN 66131; eine Veröffentlichung der BET-Methode findet sich auch in J. Am. Chem. Soc. 60,309 (1938). Die zu bestimmende Probe wurde in einem U-förmigen Quarzreaktor bei 200 °C unter Ar-Atmosphäre getrocknet (F = 50 ml(min) für 1,5 h). Der Reaktor wurde dann auf Raumtemperatur abgekühlt, evakuiert und in ein Dewar-Gefäß mit flüssigem Stickstoff getaucht. Die Stickstoff-Adsorption wurde bei 77 K mit einem RXM 100 Sorptionssystem (Advanced Scientific Design, Inc.) durchgeführt.

### Porenvolumen und Porengröße:

Das integrale Porenvolumen wurde gemäß DIN 66134, einer Bestimmung der Porengrößenverteilung und der spezifischen Oberfläche mesoporöser Feststoffe durch Stickstoffsorption gemäß BJH (Verfahren nach Barrett, Joyner und Halenda) bestimmt. Die Bestimmung des Mikro- und Meso-/Makroporenanteils wird mittels der sogenannten T-Plot Methode gemäß ASTM D-4365-85 durchgeführt.

### Ausführungsbeispiele und Vergleichsbeispiele

Ein kommerziell erhältlicher BEA-Zeolith der Süd-Chemie AG (H-BEA-150), der durch seinen hohen SiO₂/Al₂O₃-Modul von 150 stark hydrophob ist, wurde zu Kugeln mit einem Durchmesser von 3 mm verformt. Als Binder wurde dabei SiO₂ in einer Menge von 10 Gew.-% eingesetzt.

Der so erhaltene Formkörper wurde mit Silbernitrat nach dem Incipient Wetness-Verfahren imprägniert und auf einen Gehalt an elementarem Silber von 12 Gew.-% eingestellt (Adsorber A).

Der Adsorber A wurde zusätzlich mit verschiedenen Mengen an Bleinitrat nach dem Incipient Wetness-Verfahren dotiert (1 Gew.-% Pb = Adsorber B; 3 Gew.-% Pb = Adsorber C; 6 Gew.-% Pb = Adsorber D; 12 Gew.-% Pb = Adsorber E).

Vergleichsbeispiele waren 13 X Zeolithe (Hersteller SÜD-Chemie AG) mit einem Modul von 1,5, die als Kugeln gleicher Größe wie die Ausführungsbeispiele ausgebildet und mit 12 Gew.-% Silber (Adsorber F) und mit 12% Silber und 12% Blei (Adsorber G) unter Verwenden der jeweiligen Nitrate nach dem Incipient Wetness-Verfahren dotiert waren.

Zusätzlich wurde ein herkömmlicher Adsorber in Form von Aluminiumoxidkugeln gleicher Größe wie die Ausführungsbeispiele mit 12 Gew.-% Ag und silanisiert mit Propyltriethoxysilan (Dynasylan®PTEO von EVONIK) als weiteres Vergleichsbeispiel (Adsorber H) getestet.

Gemessen wurden die Methyliodidrückhaltung und die Wasserstoffbildung/-freisetzung im durchströmten Adsorberbett der Ausführungsbeispiele und Vergleichsbeispiele.

Die Messung der Rückhaltung von Methyljodid wurde in einer beheizbaren Durchflussapparatur durchgeführt. Der Durchfluss wurde auf 100 l/h eingestellt. Als Testgas wurde 200 ppmv Methyljodid in der in Tabelle 1 jeweils angegebenen Luft-Wasserdampfmischung dosiert. Für jede Messung wurden 5 g des zu untersuchenden Materials in die Apparatur eingebaut. Eingangs- und Ausgangskonzentration an Methyljodid wurde mittels FID gemessen. Als Wert für die Rückhaltung dient das Verhältnis von Ausgangskonzentration zu Eingangskonzentration nach 5 Minuten Durchströmungszeit.

Die Messung der Wasserstoffbildung/-freisetzung erfolgte wiederum in einer Durchflussapparatur. Hierbei wurde die Apparatur mit 100 l/h wasserdampfgesättigter Luft (3,2 Vol.-% H₂O) bei Raumtemperatur durchströmt. Die Apparatur besteht dabei aus zwei Segmenten mit jeweils einem Thermoelement an Ein- und Auslass. Im ersten Segment befanden sich jeweils 5,0 g der zu untersuchenden Probe. Im zweiten Segment befand sich ein platindotierter Oxidationskatalysator, der gebildeten Wasserstoff zu Wasser zurückoxidiert. Die dabei freiwerdende Reaktionswärme, die als Temperaturdifferenz zwischen Ein- und Austritt im zweiten Segment gemessen werden kann, dient als Maß für die Menge an gebildeten Wasserstoff, da sich keine weiteren, bei Raumtemperatur oxidierbaren Komponenten im Testgas befinden. 1 K Temperaturerhöhung entspricht in etwa der Bildung von 0,014 Vol.-% H₂.

Die gemessenen Methyliodidrückhaltewerte und die Menge an gebildetem Wasserstoff wurden dann mit denjenigen der Vergleichsbeispiele verglichen, wie aus den folgenden Tabellen 1 und 2 ersichtlich.

**Tabelle 1**

| **Adsorber** | **Erfindungsgemäß** | **CH₃J-Rückhaltung** | | | | | | | | |
|---|---|---|---|---|---|---|---|---|---|---|
| | | bei RT und LF von 70% | | Bei RT und 50% H₂O-Dampf | | Bei RT und 90% H₂O-Dampf | | Bei 200°C und 90% H₂O-Dampf | | |
| A | ja | > 99 % | | > 99 % | | > 99 % | | > 99 % | | |
| B | ja | > 99 % | | > 99 % | | > 99 % | | > 99 % | | |
| C | ja | > 99 % | | > 99 % | | > 99 % | | > 99 % | | |
| D | ja | > 99 % | | > 99 % | | > 99 % | | > 99 % | | |
| E | ja | > 99 % | | > 99 % | | > 99 % | | > 99 % | | |
| F | nein | 85% | | ca.80% | | ca.70% | | ca.60% | | |
| G | nein | 85% | | ca.80% | | ca.70% | | ca.60% | | |
| H | nein | 98% | | ca.90% | | ca.85% | | ca.30% | | |

**Tabelle 2**

| | Erfindungsgemäß | Wasserstofffreisetzung | | Stabilität der Hydrophobizität | |
|---|---|---|---|---|---|
| | | | Temperaturdifferenz in Reaktorsegment 2 in K | | |
| A | ja | Spuren | 3 | bis > 1000°C | |
| B | ja | Spuren | 3 | bis > 1000°C | |
| C | ja | nicht messbar | <1 | bis > 1000°C | |
| D | ja | nicht messbar | <1 | bis > 1000°C | |
| E | ja | nicht messbar | <1 | bis > 1000°C | |
| F | nein | Spuren | 2 | keine Hydrophobizität | |
| G | nein | nicht messbar | <1 | keine Hydrophobizität | |
| H | nein | nicht messbar | <1 | bis 180°C | |

| | | | | | |
|---|---|---|---|---|---|
| RT = Raumtemperatur = 25°C LF = Luftfeuchtigkeit | | | | | |

Aus Tabelle 1 ist ersichtlich, dass die Ausführungsbeispiele bei unterschiedlichen Temperaturen und Luftfeuchtigkeiten bzw. Wasserdampfkonzentrationen in der Atmosphäre eine CH₃J-Rückhaltung von mehr als 99 % zeigten, während diejenige der Vergleichsbeispiele zu weniger als 99 % CH₃J-Rückhaltung führten. Die Iodid-Adsorptionskapazität der Ausführungsbeispiele ist somit deutlich besser als diejenige der Vergleichsbeispiele.

Tabelle 2 zeigt ferner, dass alle Ausführungsbeispiele und Vergleichsbeispiele nur eine geringe bzw. nicht messbare Wasserstofffreisetzung bewirkten.

Die in den Ausführungsbeispielen eingesetzten silberhaltigen BEA-150-Zeolithe und die entsprechenden Adsorber zeichnen sich zudem durch eine hohe Temperaturbeständigkeit der Hydrophobizität bis zu mehr als 1000°C aus. Dies gilt insbesondere im Vergleich zum Vergleichsbeispiel H, das durch Silanisieren mit hydrophoben Eigenschaften versehen ist, dessen Silanschicht jedoch bereits bei 180°C thermisch zersetzbar ist.

## Patentansprüche

1. Methyliodidadsorber, umfassend einen Zeolithen, der Silber oder eine Verbindung davon enthält, wobei der Zeolith ein hydrophober Zeolith ist, der Zeolith vom Strukturtyp BEA ist und wobei der Zeolith ein SiO₂/Al₂O₃-Verhältnis > 100 besitzt, **dadurch gekennzeichnet, dass** das Silber durch Incipient Wetness-Imprägnation in den Zeolithen eingebracht ist und der Gehalt an Silber im Zeolithen 5 bis 30 Gew.-% beträgt.

2. Methyliodidadsorber nach Anspruch 1, bei dem der Zeolith oder der Methyliodidadsorber einen Anteil an Mikroporen von mehr als 70 %, bevorzugt mehr als 80 %, bezogen auf das Gesamtporenvolumen des Zeolithen oder des Methyliodidadsorbers, aufweist.

3. Methyliodidadsorber nach Anspruch 1 oder 2, bei dem mindestens ein Promotor zur Verringerung der Wasserzersetzung durch das Silber enthalten ist.

4. Methyliodidadsorber nach einem der vorstehenden Ansprüche,
bei dem der Promotor Blei umfasst; und/oder bei dem der Gehalt an Silber im Zeolithen oder im Methyliodidadsorber 10 bis 20 Gew.-%, beträgt; und/oder bei dem der Gehalt an Promotor oder Blei im Zeolithen oder im Methyliodidadsorber 1 bis 30 Gew.-%, mehr bevorzugt 5 bis 30 Gew.-%, weiter bevorzugt 10 bis 20 Gew.-%, und am meisten bevorzugt 3 bis 6 Gew.-% beträgt.

5. Methyliodidadsorber nach einem der vorstehenden Ansprüche, bei dem die BET-Oberfläche des Zeolithen oder des Methyliodidadsorbers 10 bis 1000 m²/g, bevorzugt 300 bis 900 m²/g, besonders bevorzugt 500 bis 700 m²/g beträgt und/oder das integrale Porenvolumen des Zeolithen oder des Methyliodidadsorber größer als 100 mm³/g ist.

6. Methyliodidadsorber nach einem der vorstehenden Ansprüche,
bei dem der Methyliodidadsorber als Schüttgut ausgebildet ist; und/oder
bei dem der Methyliodidadsorber einen Träger umfasst, auf dem der Zeolith oder ein den Zeolithen enthaltender Washcoat aufgebracht ist.

7. Methyliodidadsorber nach Anspruch 6,
bei dem der Methyliodidadsorber als Extrudat, als Formkörper oder als mit dem Zeolithen beschichteter Partikel ausgebildet ist; und/oder
bei dem der Methyliodidadsorber und/oder der Träger wabenförmig oder plattenförmig ausgebildet ist.

8. Verwendung des Methyliodidadsorbers nach einem der vorstehenden Ansprüche zur Adsorption von Methyliodid, radioaktivem Methyliodid, Iod und/oder radioaktivem Iod.

9. Verwendung nach Anspruch 8,
bei der der Methyliodidadsorber in oder in der Nähe von Kernkraftwerken, Wiederaufbereitungsanlagen oder Brennelementlagern eingesetzt wird; und/oder
bei der der Methyliodidadsorber zusammen mit einem Wasserstoffrekombinationskatalysator eingesetzt wird.

10. Verfahren zur Adsorption von Methyliodid, bei dem Methyliodid mit einem Methyliodidadsorber nach einem der Ansprüche 1 bis 8 in Kontakt gebracht wird.

11. Verfahren nach Anspruch 10, bei dem das Methyliodid radioaktiv ist; und/oder
bei dem zusätzlich Iod und/oder radioaktives Iod vom Methyliodidadsorber adsorbiert wird.

## Claims

1. Methyl iodide adsorber comprising a zeolite which contains silver or a compound thereof, where the zeolite is a hydrophobic zeolite, the zeolite is of the BEA structure type and wherein the zeolite has an SiO₂/Al₂O₃ ratio of > 100, **characterized in that** the silver is introduced into the zeolite by incipient wetness impregnation and the content of silver in the zeolite is from 5 to 30% by weight.

2. Methyl iodide adsorber according to Claim 1, wherein the zeolite or the methyl iodide adsorber has a proportion of micropores of more than 70%, preferably more than 80%, based on the total pore volume of the zeolite or of the methyl iodide adsorber.

3. Methyl iodide adsorber according to Claim 1 or 2, wherein at least one promoter for reducing the decomposition of water by the silver is present.

4. Methyl iodide adsorber according to any of the preceding claims,
wherein the promoter comprises lead; and/or
wherein the content of silver in the zeolite or in the methyl iodide adsorber is from 10 to 20% by weight; and/or
wherein the content of promoter or lead in the zeolite or in the methyl iodide adsorber is from 1 to 30% by weight, more preferably from 5 to 30% by weight, more preferably from 10 to 20% by weight and most preferably from 3 to 6% by weight.

5. Methyl iodide adsorber according to any of the preceding claims, wherein the BET surface area of the zeolite or of the methyl iodide adsorber is from 10 to 1000 m²/g, preferably from 300 to 900 m²/g, particularly preferably from 500 to 700 m²/g, and/or the integrated pore volume of the zeolite or of the methyl iodide adsorber is greater than 100 mm³/g.

6. Methyl iodide adsorber according to any of the preceding claims,
wherein the methyl iodide adsorber is configured as bed material; and/or
wherein the methyl iodide adsorber comprises a support to which the zeolite or a washcoat containing the zeolite has been applied.

7. Methyl iodide adsorber according to Claim 6,
wherein the methyl iodide adsorber is configured as extrudate, as shaped body or as particles coated with the zeolites; and/or
wherein the methyl iodide adsorber and/or the support is in the form of a honeycomb or a plate.

8. Use of the methyl iodide adsorber according to any of the preceding claims for the adsorption of methyl iodide, radioactive methyl iodide, iodine and/or radioactive iodine.

9. Use according to Claim 8,
wherein the methyl iodide adsorber is used in or in the vicinity of nuclear power stations, reproducing plants or fuel element stores; and/or
wherein the methyl iodide adsorber is used together with a hydrogen recombination catalyst.

10. Method for adsorbing methyl iodide, wherein the methyl iodide is brought into contact with a methyl iodide adsorber according to any of Claims 1 to 8.

11. Method according to Claim 10, wherein the methyl iodide is radioactive; and/or
wherein iodine and/or radioactive iodine is additionally adsorbed by the methyl iodide adsorber.

## Revendications

1. Adsorbant d'iodure de méthyle, comprenant une zéolithe qui contient de l'argent ou un composé de celui-ci, la zéolithe étant une zéolithe hydrophobe, qui est une zéolithe de type de structure BEA et la zéolithe présentant un rapport SiO₂/Al₂O₃ > 100, **caractérisé en ce que** l'argent est introduit par imprégnation d'humidité naissante dans la zéolithe et la teneur en argent dans la zéolithe est de 5 à 30% en poids.

2. Adsorbant d'iodure de méthyle selon la revendication 1, dans lequel la zéolithe ou l'adsorbant d'iodure de méthyle présente une proportion de micropores de plus de 70%, de préférence de plus de 80%, par rapport au volume total de pores de la zéolithe ou de l'adsorbant d'iodure de méthyle.

3. Adsorbant d'iodure de méthyle selon la revendication 1 ou 2, dans lequel est contenu un promoteur pour diminuer la décomposition de l'eau par l'argent.

4. Adsorbant d'iodure de méthyle selon l'une quelconque des revendications précédentes, dans lequel le promoteur comprend du plomb ; et/ou dans lequel la teneur en argent dans la zéolithe ou dans l'adsorbant de méthyle est de 10 à 20% en poids ; et/ou dans lequel la teneur en promoteur ou en plomb dans la zéolithe ou dans l'adsorbant d'iodure de méthyle et de 1 à 30% en poids, plus préférablement de 5 à 30% en poids, encore plus préférablement de 10 à 20% en poids et le plus préférablement de 3 à 6% en poids.

5. Adsorbant d'iodure de méthyle selon l'une quelconque des revendications précédentes, dans lequel la surface BET de la zéolithe ou de l'adsorbant d'iodure de méthyle est de 10 à 1000 m²/g, de préférence de 300 à 900 m²/g, de manière particulièrement préférée de 500 à 700 m²/g et/ou le volume intégral des pores de la zéolithe ou de l'adsorbant d'iodure de méthyle est supérieur à 100 mm³/g.

6. Adsorbant d'iodure de méthyle selon l'une quelconque des revendications précédentes, dans lequel l'adsorbant d'iodure de méthyle est conçu sous forme de matériau en vrac ; et/ou dans lequel l'adsorbant d'iodure de méthyle comprend un support sur lequel est appliqué(e) la zéolithe ou un imprégnateur contenant la zéolithe.

7. Adsorbant d'iodure de méthyle selon la revendication 6, dans lequel l'adsorbant d'iodure de méthyle est conçu sous forme de produit extrudé, sous forme de corps façonné ou sous forme de particules revêtues de la zéolithe ; et/ou dans lequel l'adsorbant d'iodure de méthyle et/ou le support est conçu sous forme de nid d'abeille ou de plaque.

8. Utilisation de l'adsorbant d'iodure de méthyle selon l'une quelconque des revendications précédentes pour l'adsorption d'iodure de méthyle, d'iodure de méthyle radioactif, d'iode et/ou d'iode radioactif.

9. Utilisation selon la revendication 8, dans laquelle l'adsorbant d'iodure de méthyle est utilisé dans ou à proximité de centrales nucléaires, d'installations de retraitement ou d'entrepôts d'éléments de combustible et/ou dans laquelle l'adsorbant d'iodure de méthyle est utilisé conjointement avec un catalyseur de recombinaison d'hydrogène.

10. Procédé pour l'adsorption d'iodure de méthyle, dans lequel de l'iodure de méthyle est mis en contact avec un adsorbant d'iodure de méthyle selon l'une quelconque des revendications 1 à 8.

11. Procédé selon la revendication 10, dans lequel l'iodure de méthyle est radioactif ; et/ou dans lequel de l'iode et/ou de l'iode radioactif est adsorbé en plus par l'adsorbant d'iodure de méthyle.
